# EUROPÄISCHE PATENTSCHRIFT

(11) **EP 4 046 583 B1**
(45) Veröffentlichungstag und Bekanntmachung des Hinweises auf die Patenterteilung: **25.03.2026**
(21) Anmeldenummer: 22156720.9
(22) Anmeldetag: 15.02.2022
(51) Int. Cl.: A61B 18/14, A61B 18/00

(54) **ELEKTROCHIRURGISCHES HANDGERÄT UND KONTAKTKÖRPER FÜR ELEKTROCHIRURGISCHES HANDGERÄT**
ELECTROSURGICAL INSTRUMENT AND CONTACT BODY FOR ELECTROSURGICAL INSTRUMENT
APPAREIL ÉLECTROCHIRURGICAL PORTATIF ET CORPS DE CONTACT POUR APPAREILS ELECTROCHIRURGICAUX PORTATIFS

(30) Priorität: 22.02.2021 DE 102021104119
(43) Veröffentlichungstag der Anmeldung: 24.08.2022
(73) Patentinhaber: Olympus Winter & Ibe GmbH, 22045 Hamburg (DE)
(72) Erfinder: Offt, Andreas, 21465 Reinbek (DE); Brockmann, Christian, 21279 Hollenstedt (DE); Torkuhl, Nils, 24802 Groß Vollstedt (DE); Drechsler, Dorian, 22147 Hamburg (DE)
(74) Vertreter: Hoener, Matthias

(56) Entgegenhaltungen:
- WO-A1-95/19142
- US-A1- 2001 011 161

## Beschreibung

Die Erfindung betrifft einen Kontaktkörper für elektrochirurgische Handgeräte gemäß dem Oberbegriff des Anspruchs 1. Außerdem betrifft die Erfindung ein elektrochirurgisches Handgerät gemäß dem Anspruch 11.

Elektrochirurgische Handgeräte, wie beispielsweise Resektoskope, werden hauptsächlich für endoskopische Anwendungen in der Urologie und Gynäkologie eingesetzt und dort vorzugsweise für die Behandlung im Bereich der Blase, Gebärmutter oder der Prostata. Das Einsatzgebiet dieser Instrumente ist jedoch nicht auf diese Bereiche des menschlichen Körpers beschränkt, sondern umfasst vielmehr auch die Behandlung weiterer Organe im menschlichen Unterleib.

Die Dokumente US2001/011161A1 und WO95/19142A1 offenbaren bekannte elektrochirurgische Geräte.

Die hier beschriebenen gattungsgemäßen Instrumente, wie beispielsweise Resektoskope, weisen standardmäßig einen Transporteur auf. Zur Behandlung des erkrankten Gewebes wird das Resektoskop mit einem langgestreckten Schaft durch eine Öffnung in den Körper des Patienten eingeführt. In diesem Schaftrohr können verschiedene medizinische Instrumente zur Behandlung und/oder zur Untersuchung des Patienten angeordnet sein. Zum Beispiel kann etwa für die Hochfrequenzchirurgie eine mit hochfrequentem Wechselstrom beaufschlagbare Elektrode, die an einem distalen Ende eines Elektrodenträgers positioniert ist, in das Schaftrohr eingeführt werden. Für am Patienten durchzuführende Behandlungen, wie beispielsweise das Schneiden von erkranktem Gewebe, ist der Elektrodenträger mit der Elektrode relativ zum Schaftrohr sowie entlang einer Schaftachse verschiebbar an dem Resektoskop angeordnet. Dabei ist die Elektrode bzw. das Werkzeug dem distalen Ende des Schaftrohres zugeordnet.

Der Elektrodenträger ist des Weiteren mit seinem proximalen Ende an den Transporteur gekoppelt, von dem er entlang der Schaftachse verschoben werden kann. Dadurch wird die Schneidbewegung der Elektrode ermöglicht. Der Transporteur ist üblicherweise lösbar mit dem Schaftrohr gekoppelt. Er weist einen bewegbar gelagerten Kontaktkörper auf, der auch als Schlitten bezeichnet wird. An diesem Kontaktkörper kann der Elektrodenträger mit mindestens einem elektrischen Kontakt mechanisch lösbar gekoppelt sein. Über diese mechanische Verbindung ist der Elektrodenträger bzw. die Elektrode auch mit elektrischer Energie versorgbar. Dazu weißt der Kontaktkörper mindestens eine Öffnung auf, in die mindestens ein elektrischer Kontakt des Elektrodenträgers für die lösbare Verbindung führbar ist. Diese Öffnung bzw. dieses Sackloch oder diese Bohrung ist derart ausgebildet, dass über eine angrenzende Steckerbuchse die HF-Spannung beaufschlagbar ist. Dazu wird üblicherweise ein Stecker in die Buchse gesteckt, der wiederum über eine Leitung bzw. ein Kabel mit einem HF-Generator verbindbar ist.

Die Betätigung bzw. der Längsverschub des Transporteurs wird durch einen Operateur ausgeführt. Dazu ist dem Transporteur eine Griffeinheit mit einem ersten Griffmittel und einem zweiten Griffmittel zugeordnet. Der Operateur erfasst zum Betätigen des Transporteurs das erste Griffmittel, als auch ein zweites Griffmittel, welches über eine Fingereinheit oder einen Daumenring verfügen kann. Das erste Griffmittel kann an einem feststehenden Hauptkörper des Transporteurs befestigt sein. Das zweite Griffmittel kann an dem Kontaktkörper befestigt sein.

Die Bewegung des Transporteurs erfolgt gegen eine Federspannkraft einer Feder, die beim gattungsgemäßen Transporteur üblicherweise als Blattfeder oder als Schenkelfeder ausgebildet ist. Diese Feder ist mit einem Ende an dem Kontaktkörper bzw. dem Schlitten und mit dem anderen Ende an einem Endkörper bzw. einer Optikführungsplatte eines Verstärkungsrohres befestigt. Die Art der Federn bzw. die Art der Betätigung dieses Federmechanismus richtet sich danach, ob es sich bei dem Transporteur um einen aktiven oder einen passiven Transporteur handelt. Während die Feder bei einem aktiven Transporteur als Druckfeder ausgebildet ist, ist sie bei einem passiven Transporteur als Zugfeder ausgebildet.

Üblicherweise erfolgt das Schneiden der Elektrode durch eine rückziehende Bewegung des Transporteurs. Beim aktiven Transporteur wird dazu die Elektrode gegen die Federkraft der Feder zurückgezogen (in proximaler Richtung). Beim passiven Transporteur hingegen wird die Elektrode zunächst entgegen der Federkraft vorgeschoben (in distale Richtung), um dann bei der durch die Entlastung der Feder hervorgerufenen Rückbewegung (in proximaler Richtung) durch das Gewebe zu schneiden.

Durch den Schaft der hier beschriebenen Instrumente ist außerdem eine Optik führbar. Die stabartige bzw. schaftartige Optik wird von dem proximalen Ende her durch ein Verstärkungsrohr, auch Optikführungsrohr genannt, des Transporteurs in den Schaft geführt. Es sind Ausführungsbeispiele bekannt, bei denen die Optik als Stablinsensystem oder als Glasfaser von einem proximalen Ende durch den Schaft zum distalen Ende geführt wird. Das distale Ende der Optik ist direkt auf den zu operierenden Bereich bzw. den Wirkort der Elektrode gerichtet. Am proximalen Ende der Optik kann der Operateur durch ein Okular oder eine Kamera die Behandlung beobachten.

Das Elektrodensystem wird mit dem mindestens einen elektrischen Kontakt des Kontaktkörpers verbunden und sodann beides zusammen in bzw. durch den Schaft geschoben. Durch Betätigung des zweiten Griffmittels relativ zu dem ersten Griffmittel lässt sich so der Kontaktkörper zusammen mit dem Elektrodenträger über das Verstärkungsrohr entlang der Schaftachse vor und zurück bewegen.

Bei bekannten Systemen wird das Verstärkungsrohr bei der Fertigung fest mit dem Transporteur bzw. dem Hauptkörper verbunden. Am proximalen Ende des Verstärkungsrohres ist dieses mit der Optikführungsplatte verschweißt. Vor der Verbindung des Verstärkungsrohres mit dem Hauptkörper ist über das Verstärkungsrohr der Kontaktkörper zu schieben. Dazu weist der Kontaktkörper eine entsprechende Bohrung parallel zu der Schaftachse auf. Diese Bohrung ist derart bemessen, dass sich der Kontaktkörper bzw. der Schlitten leicht über das Verstärkungsrohr schieben lässt. Als besonders nachteilig hat sich nicht nur die aufwendige Fertigung des Transporteurs erwiesen, sondern auch der erhöhte Aufwand für die Instandsetzung und der Tausch von Komponenten. Insbesondere für einen wartungsbedingten oder defektbedingten Austausch des Kontaktkörpers ist das Verstärkungsrohr von dem Hauptkörper aufwendig zu entfernen, um den Kontaktkörper vom selbigen abziehen zu können.

Davon ausgehend liegt der Erfindung die Aufgabe zu Grunde, ein elektrochirurgisches Handgerät sowie einen Kontaktkörper zu schaffen, der besonders einfach sowie zeiteffizient handhabbar bzw. wartbar ist.

Ein Kontaktkörper zur Lösung dieser Aufgabe weist die Merkmale des Anspruchs 1 auf. Demnach ist vorgesehen, dass ein Kontaktkörper für ein elektrochirurgisches Handgerät parallel zu einer durchgängigen Bohrung sowie parallel zu einer Längsachse des Handgerätes einen Schlitz aufweist. Die Bohrung ist zur Aufnahme einer Optikführung vorgesehen. Diese Optikführung kann beispielsweise als Verstärkungsrohr oder als rohrartiger Schaft zur Aufnahme einer stabartigen Optik ausgebildet sein. Die Bohrung erstreckt sich von einer Stirnseite des Kontaktkörpers zu einer gegenüberliegenden Stirnseite, wobei die Bohrung parallel zu einer Längsachse des elektrochirurgischen Handgerätes ausgerichtet ist.

Der Schlitz ist erfindungsgemäß derart ausgebildet, dass sich der Kontaktkörper über die rohrartige Optikführung stecken lässt, wobei die Optikführung durch den Schlitz in die Bohrung bewegt wird. Gleichermaßen ist es selbstverständlich auch denkbar, dass sich die rohrartige Optikführung durch den Schlitz in die Bohrung drücken lässt. Bedingt durch den Schlitz lässt sich der Kontaktkörper somit auch an ein wenigstens nahezu fertig montiertes Handgerät bzw. einen Transporteur des Handgerätes befestigen. Gleichermaßen kann der Kontaktkörper für Wartungsarbeiten von der Optikführung gelöst werden und zwar ohne, dass dazu eine Demontage des Handgerätes größeren Umfanges nötig wäre. Durch den erfindungsgemäßen Kontaktkörper lassen sich die Herstellung und die Wartung besonders einfach sowie zeiteffizient durchführen.

Insbesondere kann es die Erfindung weiter vorsehen, dass sich der Schlitz durch den Kontaktkörper von einer Außenwandung des Kontaktkörpers bis zu der Bohrung erstreckt, wobei die rohrartige Optikführung durch den Schlitz in die Bohrung führbar ist. Der Schlitz und die Bohrung bilden somit zusammen eine Ausnehmung in dem Kontaktkörper. Letztendlich stellt der Schlitz eine Erweiterung des Innenraumes der Bohrung dar. Durch diese Erweiterung kann der Bohrung die Optikführung auf eine einfache Weise zugeführt als auch wieder entnommen werden. Sofern die schlitzartige Ausgestaltung der Erweiterung nicht zu breit ist, ändert sich an der gleitenden Verbindung zwischen der Optikführung und einer Innenwandung der Bohrung nichts. Vielmehr bleibt die Handhabung des Handgerätes durch diesen Schlitz unbeeinflusst.

Vorzugsweise ist es darüber hinaus denkbar, dass der Schlitz zwei parallele oder einen Winkel einschließende Seitenwandungen aufweist oder dass die Seitenwandungen einen dreieckigen Querschnitt aufweisen, wobei sich zwei Ecken der Seitenwände parallel zueinander direkt gegenüber liegen. Durch diese Ausgestaltungen der Seitenwandungen wird zum einen sichergestellt, dass die Optikführung sowohl in die Bohrung fügbar ist als auch wieder aus dieser entnehmbar ist. Darüber hinaus wird sichergestellt, dass die Optikführung nicht ungewollt aus der Bohrung durch den Schlitz herausrutscht.

Bevorzugt ist es weiter denkbar, dass ein Querschnitt des Kontaktkörpers eine ringförmige, vorzugsweise kreisartige oder ovale, Form aufweist, wobei der Schlitz eine Öffnung der Form darstellt. Durch diese ringartige Ausgestaltung ist es denkbar, dass die offenen den Schlitz bildenden Ringenden reversibel verformbar sind, um während der Aufnahme der Optikführung wenigstens vorübergehend derart auseinander bewegt zu werden, dass die Optikführung in die Bohrung führbar ist. Sobald die Optikführung in der Bohrung platziert ist, kann der Kontaktkörper seine ursprüngliche Form wieder annehmen. Gleichermaßen ist es denkbar, dass sich der Kontaktkörper nicht verformt, sondern vielmehr ein Querschnitt der rohrartigen Optikführung wenigstens vorübergehend veränderbar ist, um durch den Schlitz in die Bohrung geführt zu werden.

Ein weiteres vorteilhaftes Ausführungsbeispiel der Erfindung kann es vorsehen, dass eine Ebene, die sich parallel sowie mittig zwischen den beiden Seitenwänden des Schlitzes erstreckt eine Mittelachse der Bohrung schneidet. Durch diese relative Ausrichtung der Seitenwände und der Bohrung lässt sich die Optikführung auf eine besonders einfache Art und Weise in die Bohrung führen. Darüber hinaus ist es denkbar, dass die vorgenannte Ebene leicht von der Mittelachse entrückt ist. Dadurch kann das Einführen bzw. das Herausnehmen der Optikführung aus der Bohrung erleichtert werden.

Ein besonders vorteilhaftes Ausführungsbeispiel der Erfindung sieht es vor, dass eine Weite, sprich ein Abstand der Seitenwandungen, des Schlitzes geringer ist als der Durchmesser der Bohrung. Vorzugsweise kann die Bohrung einen Durchmesser von 3 mm bis 6 mm, bevorzugt 4 mm bis 5 mm, insbesondere 4,6 mm, aufweisen und der Schlitz eine Weite von 2 mm bis 5 mm, vorzugsweise 3 mm bis 4 mm, insbesondere 3,5 mm, aufweisen. Der Durchmesser der Bohrung ist dabei stets geringfügig größer als der Durchmesser der rohrartigen Optikführung.

Als besonders bevorzugt hat sich ein Verhältnis zwischen einer Weite des Schlitzes, insbesondere eines geringsten Abstandes der Seitenwandungen des Schlitzes, und einem Durchmesser der Bohrung von 0,6 - 0,9, vorzugsweise 0,7 - 0,8, insbesondere 0,76, erwiesen. Dieses Verhältnis der Weite und des Durchmessers ist besonders bevorzugt für ein einfaches Einführen der Optikführung als auch ein ausreichend großer Geleitswiderstand des Kontaktkörpers auf der Optikführung. Bei einem zu kleinem Verhältnis besteht insbesondere das Risiko, die Bauteile plastisch zu verformen. Bei einem zu großem Verhältnis ist die Führung nicht mehr gewährleistet. Die beschriebenen Werte gelten für PTFE und können bei anderen Werkstoffen abweichen

Der hier beschriebene Kontaktkörper des chirurgischen Handgerätes kann auch als Schlitten eines aktiven oder passiven Resektoskopes ausgebildet sein. Es hat sich herausgestellt, dass Kunststoff, insbesondere PTFE, für den Kontaktkörper aufgrund der Materialeigenschaften wie beispielsweise geringer Geleitswiderstand, hoher elektrischer Widerstand, glatte Oberfläche sowie gute Bearbeitbarkeit besonders vorteilhaft ist. Gleichermaßen ist es jedoch auch denkbar, dass der Kontaktkörper aus einem anderen Fluorpolymer wie beispielsweise PFA herstellbar ist. Auch PEEK hat sich als vorteilhaft erwiesen.

Weiter ist es erfindungsgemäß denkbar, dass der Kontaktkörper mindestens eine Aufnahme für einen Kontakt des Elektroneninstrumentes aufweist und diese Aufnahme mit mindestens einer Steckerbuchse verbindbar ist, insbesondere das in den Kontaktkörper einer Steckerbuchse integriert ist. Wenn das chirurgische Handgerät ein Elektrodeninstrument mit lediglich einem elektrischen Kontakt aufweist, weist der Kontaktkörper ebenfalls nur eine entsprechende Aufnahme auf. Wenn das Elektrodeninstrument allerdings zwei Kontakte aufweist, beispielsweise einen Aktiv-Kontakt und einen Return-Kontakt, so kann der Kontaktkörper dementsprechend zwei Aufnahmen für die elektrische Kontaktierung aufweisen. Diese Aufnahmen sind ebenfalls parallel zu der Bohrung in dem Kontaktkörper angeordnet und können sacklochartig ausgebildet sein oder sich durch den gesamten Körper hindurch erstrecken.

Ein elektrochirurgisches Handgerät zur Lösung der eingangs genannten Aufgabe weist die Merkmale des Anspruchs 11 auf. Demnach ist es vorgesehen, dass das Handgerät, bei dem es sich vorzugsweise um ein Resektoskop mit einem aktiven oder passiven Transporteur handeln kann, ein Elektrodeninstrument aufweist, das an einem distalen Ende eine Elektrode aufweist und an einem proximalen Ende mindestens einen elektrischen Kontakt. Außerdem weist das Handgerät eine Griffeinheit auf mit einem ersten Griffmittel und einem zweiten Griffmittel. Darüber hinaus weist das Instrument einen rohrartigen Schaft auf, der mit einem proximalen Ende an das erst ersten Griffmittel gekoppelt ist und eine Optikführung zur Aufnahme einer Optik. Diese Optikführung ist durch einen Kontaktkörper führbar, wobei an diesem Kontaktkörper auch das zweite Griffmittel sowie eine Feder befestigt ist und mindestens eine Aufnahme für einen elektrischen Kontakt des Elektroneninstrumentes aufweist. Dieser Kontaktkörper ist erfindungsgemäß nach mindestens einem der vorherigen Ansprüche ausgebildet.

Ein bevorzugtes Ausführungsbeispiel der Erfindung wird im Folgenden anhand der Zeichnung näher erläutert. In dieser zeigen:
- Fig. 1: eine schematische Darstellung eines Resektoskopes,
- Fig. 2: eine schematische Darstellung eines Transporteurs,
- Fig. 3: eine perspektivische Darstellung einer Optikführung,
- Fig. 4: eine perspektivische Darstellung eines Kontaktkörpers, und
- Fig. 5: eine Sicht auf eine Stirnseite des Kontaktkörpers gemäß Fig. 4.

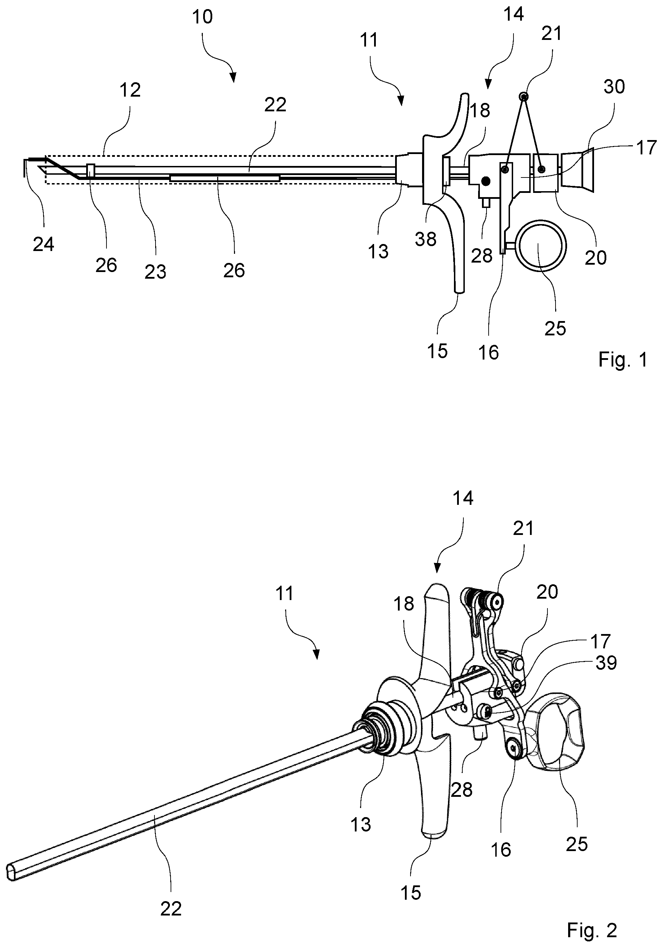

Ein mögliches Ausführungsbeispiel eines elektrochirurgischen Handgerätes, nämlich eines Resektoskopes 10, ist stark schematisiert in der Fig. 1 dargestellt. Das Resektoskop 10 weist einen Transporteur 11 (siehe auch Fig. 2) auf, an dem ein langgestreckter, rohrartiger Schaft 12 befestigbar ist. Dieser Schaft 12 ist in der Fig. 1 schraffiert dargestellt und ist mit einem proximalen Ende an einem Hauptkörper 13 des Transporteurs 11 befestigt.

Der Transporteur 11 weist neben dem Hauptkörper 13 eine Griffeinheit 14 auf. Diese Griffeinheit 14 verfügt über ein erstes Griffmittel 15 und ein zweites Griffmittel 16. Während das erste Griffmittel 15 fest an dem Hauptkörper 13 angeordnet ist, ist bei dem hier dargestellten Ausführungsbeispiel des Transporteurs 11 das zweite Griffmittel 16 einem Kontaktkörper 17 zugeordnet. Dabei ist es denkbar, dass das zweite Griffmittel 16 an dem Kontaktkörper 17 festgeschraubt ist. Dazu weist der Kontaktkörper 17 in einer Wandung eine entsprechende Bohrung 31 auf.

Der Kontaktkörper 17 wird gleitend auf einer rohrartigen Optikführung 18 geführt. Dazu weist der Kontaktkörper 17 eine Bohrung 19 auf, deren Durchmesser geringfügig größer ist als ein Durchmesser der Optikführung 18. Da sich der Kontaktkörper 17 auf der Optikführung 18 entlang einer Längsrichtung des Resektoskops 10 bzw. einer Längsachse des Schaftes 12 hin und her bewegen lässt, wird der Kontaktkörper 17 auch als Schlitten bezeichnet.

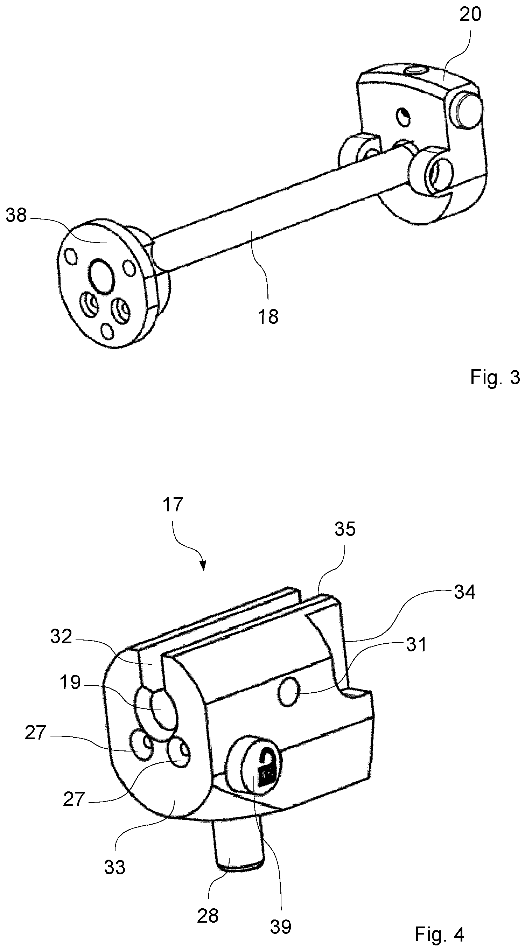

Während die Optikführung 18 mit einem distalen Ende über einen Adapter 38 (Fig. 3) mit dem Hauptkörper 13 oder einem Innenrohr 22 verbunden ist, ist an einem proximalen Ende der Optikführung 18 eine Optikführungsplatte 20 befestigt. Die rohrartige Optikführung 18 erstreckt sich durch die Optikführungsplatte 20, sodass die Optikführung 18 von proximal aus zugänglich ist.

Das zweite Griffmittel 16 bzw. der Kontaktkörper 17 sind über ein Federelement 21 mit der Optikführungsplatte 20 verbunden. Bei diesem Federelement 21 kann es um eine Zugfeder handeln.

Ausgehend von dem Hauptkörper 13 erstreckt sich ein Innenrohr 22 in distale Richtung. Dieses Innenrohr 22 kann sich auch in proximale Richtung durch den Hauptkörper 13 erstrecken und mit der Optikführung 18 verbunden werden. Gleichermaßen ist es denkbar, dass das Innenrohr 22 und die Optikführung 18 einteilig ausgebildet sind bzw. dass sich die Optikführung 18 durch den Hauptkörper 13 nach distal erstreckt.

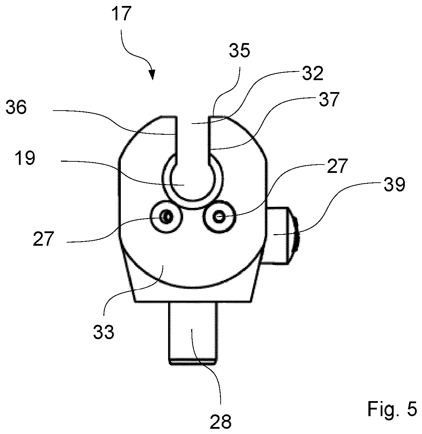

Parallel zu dem Innenrohr 22 erstreckt sich ein Elektrodeninstrument 23. Dieses Elektrodeninstrument 23 wird durch den Hauptkörper 13 geführt und ist mit wenigstens einem proximalen Kontakt mechanisch in einer Aufnahme 27 mit dem Kontaktkörper 17 lösbar gekoppelt. In dem Kontaktkörper 17 kann ein Rastmechanismus vorgesehen sein, der über einen Knopf 39 (Fig. 4, 5) lösbar und feststellbar ist. Der Rastmechanismus arretiert das wenigstens eine proximale Ende bzw. den Kontakt des Elektrodeninstrumentes 23 in dem Kontaktkörper 17. Der Knopf 39 bzw. der Rastmechanismus kann federvorgespannt sein und ist einfach mit einem Finger betätigbar.

An einem distalen Ende weist das Elektrodeninstrument 23 eine Elektrode 24 auf. Diese Elektrode 24 ist mit einer elektrischen HF-Spannung beaufschlagbar. Mittels eines sich an der Elektrode 24 bildenden Plasmas lässt sich das erkrankte Gewebe manipulieren bzw. schneiden. Dazu bewegt der Operateur das einen Daumenring 25 aufweisende zweite Griffmittel 16 relativ zu dem ersten Griffmittel 15. Zur Stabilisierung des Elektrodeninstrumentes 23 kann dieses an dem Innenrohr 22 durch Führungen 26 geführt werden.

Für die Beaufschlagung der Elektrode 24 mit der HF-Spannung lässt sich die Aufnahme 27 des proximalen Kontaktes des Elektrodeninstrumentes 23 elektrisch kontaktieren. Dazu weist der Kontaktkörper 17 mindestens eine Steckerbuchse 28 (Fig. 4, 5) auf. Diese Steckerbuchse 28 befindet sich im elektrischen Kontakt mit wenigstens einem Teil einer Innenwandung der Aufnahme 27. Über einen hier nicht dargestellten Stecker lässt sich so der Kontaktkörper 17 über ein Kabel mit einem HF-Generator verbinden.

Für die Durchführung des Eingriffs wird durch das Innenrohr 22 bzw. Optikführung 18 eine stabartige Optik 29 geführt. Ein hier nicht sichtbares distales Ende dieser Optik 29 ist in Richtung der Elektrode 24 gerichtet, damit der Operateur die Manipulation des Gewebes im Blick hat. Bei dieser Optik 29 kann es sich um ein Stablinsensystem oder um eine Glasfaser handeln. Am proximalen Ende der Optik 29 befindet sich, wie in der Fig. 1 dargestellt, ein Okular 30 oder eine Kamera.

Bei der Fertigung des Transporteurs 11 erweist sich die Montage des Kontaktkörpers 17 als besonders umständlich. Bisher war es so, dass zunächst die Optikführungsplatte 20 an der Optikführung 18 verschweißt wurde, dann der Kontaktkörper 17 auf die Optikführung 18 gesteckt wurde und sodann die Optikführung 18 mit dem distalen Ende an dem Hauptkörper 13 oder dem Innenrohr 22 fest verbunden wurde. Zum Austausch oder zu Wartungszwecken des Kontaktkörpers 17 mussten diese Schritte in umgekehrter Reihenfolge wiederholt werden.

Der hier dargestellte Kontaktkörper 17 weist einen Schlitz 32 auf (Fig. 4). Dieser Schlitz 32 erstreckt sich parallel zu der Bohrung 19 von einer Stirnseite 33 zu der gegenüberliegenden Stirnseite 34 des Kontaktkörpers 17. Der Schlitz 32 ist derart ausgebildet, dass er sich von einer Außenwandung 35 bis zu der Bohrung 19 erstreckt (Fig. 5). Dadurch wird der Innenraum der Bohrung 19 erweitert.

Bei dem in der Fig. 5 dargestellten Ausführungsbeispiel des Schlitzes 32 weist dieser zwei parallele Seitenwandungen 36, 37 auf. Der Abstand dieser beiden Seitenwandungen 36, 37, sprich die Weite des Schlitzes 32, ist geringer als der Durchmesser der Bohrung 19. Dabei ist es insbesondere vorgesehen, dass ein Verhältnis zwischen der Weite des Schlitzes 32 und dem Durchmesser der Bohrung 19 0,6 - 0,9, vorzugsweise 0,7 - 0,8, insbesondere 0,76, beträgt.

Durch die Erweiterung der Bohrung 19 durch den Schlitz 32 ist es möglich, den Kontaktkörper 17 auf die Optikführung 18 zu clipsen. Dazu wird die rohrartige Optikführung 18 durch den Schlitz 32 in die Bohrung 19 geführt. Dabei es ist denkbar, dass sich ein Außendurchmesser der Optikführung 18 oder der Abstand der beiden Seitenwandungen 36, 37 kurzzeitig sowie reversibel verformt. Nach der Montage des Kontaktkörpers 17 lässt sich sodann das Griffmittel 16, das Federelement 21 und das Elektrodeninstruments 23 mit dem Kontaktkörper 17 verbinden. Sofern erforderlich, können die vorgenannten Komponenten des Transporteurs 11 auf schnelle sowie einfache Art und Weise von dem Kontaktkörper 17 gelöst werden, um den Kontaktkörper 17 für Wartungszwecke sodann von der Optikführung 18 abzuziehen.

### Bezugszeichenliste

- 10: Resektoskop
- 11: Transporteur
- 12: Schaft
- 13: Hauptkörper
- 14: Griffeinheit
- 15: erstes Griffmittel
- 16: zweites Griffmittel
- 17: Kontaktkörper
- 18: Optikführung
- 19: Bohrung
- 20: Optikführungsplatte
- 21: Federelement
- 22: Innenrohr
- 23: Elektrodeninstrument
- 24: Elektrode
- 25: Daumenring
- 26: Führung
- 27: Aufnahme
- 28: Steckerbuchse
- 29: Optik
- 30: Okular
- 31: Bohrung
- 32: Schlitz
- 33: Stirnseite
- 34: Stirnseite
- 35: Außenwandung
- 36: Seitenwandung
- 37: Seitenwandung
- 38: Adapter
- 39: Knopf

## Patentansprüche

1. Kontaktkörper (17) für ein elektrochirurgisches Handgerät, insbesondere Resektoskop (10), für die Aufnahme einer rohrartigen Optikführung (18), für die Befestigung einer Griffeinheit (14) und zur Kopplung mindestens eines elektrischen Kontaktes eines Elektrodeninstrumentes (23) des Handgerätes, **dadurch gekennzeichnet, dass** der Kontaktkörper (17) parallel zu einer durchgängigen Bohrung (19) zur Aufnahme der Optikführung (18) sowie parallel zu einer Längsachse des Handgerätes einen Schlitz (32) aufweist, wobei der Schlitz (32) sich von einer Außenwandung (35) des Kontaktkörpers (17) bis zu der Bohrung (19) erstreckt und wobei die rohrartige Optikführung (18) durch den Schlitz (32) in die Bohrung (19) führbar ist.

2. Kontaktkörper (17) für ein elektrochirurgisches Handgerät nach Anspruch 1, **dadurch gekennzeichnet, dass** der Schlitz (32) zwei parallele oder einen Winkel einschließende Seitenwandungen (36, 37) aufweist oder dass die Seitenwandungen (36, 37) einen dreieckigen Querschnitt aufweisen, wobei sich zwei Ecken der Seitenwände (36, 37) parallel zueinander direkt gegenüberliegen.

3. Kontaktkörper (17) für ein elektrochirurgisches Handgerät nach einem der vorangegangenen Ansprüche, **dadurch gekennzeichnet, dass** ein Querschnitt des Kontaktkörpers (17) eine ringförmige, vorzugsweise kreisartige oder ovale, Form aufweist, wobei der Schlitz (32) eine Öffnung der Form darstellt.

4. Kontaktkörper (17) für ein elektrochirurgisches Handgerät nach einem der vorangegangenen Ansprüche, **dadurch gekennzeichnet, dass** eine Ebene, die sich parallel sowie mittig zwischen den Seitenwänden (36, 37) erstreckt, eine Mittelachse der Bohrung (19) schneidet.

5. Kontaktkörper (17) für ein elektrochirurgisches Handgerät nach einem der vorangegangenen Ansprüche, **dadurch gekennzeichnet, dass** eine Weite des Schlitzes (32) geringer als der Durchmesser der Bohrung (19) ist.

6. Kontaktkörper (17) für ein elektrochirurgisches Handgerät nach einem der vorangegangenen Ansprüche, **dadurch gekennzeichnet, dass** die Bohrung (19) einen Durchmesser von 3 mm bis 6 mm, vorzugsweise von 4 mm bis 5 mm, insbesondere 4,6 mm und der Schlitz (32) eine Weite von 3 mm bis 4 mm, vorzugsweise von 3,5 mm, aufweist, wobei der Durchmesser geringfügig größer ist als ein Durchmesser der rohrartigen Optikführung (18).

7. Kontaktkörper (17) für ein elektrochirurgisches Handgerät nach einem der vorangegangenen Ansprüche, **dadurch gekennzeichnet, dass** ein Verhältnis zwischen einer Weite des Schlitzes (32), insbesondere ein Abstand der Seitenwandungen (36, 37) des Schlitzes (32), und einem Durchmesser der Bohrung (19) 0,6 bis 0,9, vorzugsweise 0,7 bis 0,8, insbesondere 0,76, beträgt.

8. Kontaktkörper (17) für ein elektrochirurgisches Handgerät nach einem der vorangegangenen Ansprüche, **dadurch gekennzeichnet, dass** es sich bei dem Kontaktkörper (17) um einen Schlitten für ein aktives oder passives Resektoskop (10) handelt.

9. Kontaktkörper (17) für ein elektrochirurgisches Handgerät nach einem der vorangegangenen Ansprüche, **dadurch gekennzeichnet, dass** der Kontaktkörper (17) aus Kunststoff, insbesondere PTFE, PFA, einem anderen Fluorpolymer oder PEEK hergestellt ist.

10. Kontaktkörper (17) für ein elektrochirurgisches Handgerät nach einem der vorangegangenen Ansprüche, **dadurch gekennzeichnet, dass** der Kontaktkörper (17) mindestens eine Aufnahme (27) für einen Kontakt des Elektrodeninstrumentes (23) aufweist und diese Aufnahme (27) mit mindestens einer Steckerbuchse (28) verbunden ist, insbesondere dass in den Kontaktkörper (17) eine Steckerbuchse (28) integriert ist.

11. Elektrochirurgisches Handgerät, insbesondere Resektoskop (10), mit einem Elektrodeninstrument (23), das an einem distalem Ende eine Elektrode (24) aufweist und an einem proximalen Ende mindestens einen elektrischen Kontakt mit einer Griffeinheit (14) bestehend aus einem ersten Griffmittel (15) und einem zweiten Griffmittel (16), mit einem rohrartigen Schaft (12), der mit einem proximalen Ende an dem ersten Griffmittel (15) gekoppelt ist, mit einer Optikführung (18) zur Aufnahme einer Optik (29) und einem Kontaktkörper (17) durch den die Optikführung (18) führbar ist, das zweite Griffmittel (16) befestigbar ist und in dem der mindestens eine elektrische Kontakt des Elektrodeninstrumentes (23) verrastbar und/oder elektrisch kontaktierbar ist, **gekennzeichnet durch** einen Kontaktkörper (17) nach mindestens einem der Ansprüche 1 bis 10.

## Claims

1. A contact body (17) for an electrosurgical handheld device, in particular a resectoscope (10), for receiving a tubular optical guide (18), for fastening a grip unit (14), and for coupling at least one electrical contact of an electrode instrument (23) of the handheld device, **characterized in that** the contact body (17) has a slit (32) parallel to a continuous bore (19) for receiving the optical guide (18) and also parallel to a longitudinal axis of the handheld device, wherein the slit (32) extends from an outer wall (35) of the contact body (17) as far as the bore (19) and wherein the tubular optical guide (18) can be guided through the slit (32) into the bore (19).

2. The contact body (17) for an electrosurgical handheld device as claimed in claim 1, **characterized in that** the slit (32) has two side walls (36, 37), which are parallel or enclose an angle, or **in that** the side walls (36, 37) have a triangular cross section, wherein two corners of the side walls (36, 37) lie directly opposite and parallel to each other.

3. The contact body (17) for an electrosurgical handheld device as claimed in one of the preceding claims, **characterized in that** a cross section of the contact body (17) has an annular, preferably circular or oval shape, wherein the slit (32) constitutes an opening in the shape.

4. The contact body (17) for an electrosurgical handheld device as claimed in one of the preceding claims, **characterized in that** a plane extending parallel and centrally between the side walls (36, 36) intersects a central axis of the bore (19).

5. The contact body (17) for an electrosurgical handheld device as claimed in one of the preceding claims, **characterized in that** a width of the slit (32) is less than the diameter of the bore (19).

6. The contact body (17) for an electrosurgical handheld device as claimed in one of the preceding claims, **characterized in that** the bore (19) has a diameter of 3 mm to 6 mm, preferably of 4 mm to 5 mm, in particular 4.6 mm, and the slit (32) has a width of 3 mm to 4 mm, preferably of 3.5 mm, wherein the diameter is slightly greater than a diameter of the tubular optical guide (18).

7. The contact body (17) for an electrosurgical handheld device as claimed in one of the preceding claims, **characterized in that** a ratio between a width of the slit (32), in particular a spacing of the side walls (36, 37) of the slit (32), and a diameter of the bore (19) is 0.6 to 0.9, preferably 0.7 to 0.8, in particular 0.76.

8. The contact body (17) for an electrosurgical handheld device as claimed in one of the preceding claims, **characterized in that** the contact body (17) is a slide for an active or passive resectoscope (10).

9. The contact body (17) for an electrosurgical handheld device as claimed in one of the preceding claims, **characterized in that** the contact body (17) is made of plastic, in particular PTFE, PFA, another fluoropolymer or PEEK.

10. The contact body (17) for an electrosurgical handheld device as claimed in one of the preceding claims, **characterized in that** the contact body (17) has at least one receptacle (27) for a contact of the electrode instrument (23), and this receptacle (27) is connected to at least one plug socket (28), in particular **in that** a plug socket (28) is integrated in the contact body (17).

11. An electrosurgical handheld device, in particular a resectoscope (10), with an electrode instrument (23) which at a distal end has an electrode (24) and at a proximal end has at least one electrical contact with a grip unit (14) consisting of a first gripping means (15) and a second gripping means (16), with a tubular shaft (12) which is coupled with a proximal end to the first gripping means (15), with an optical guide (18) for receiving an optical unit (29), and a contact body (17) through which the optical guide (18) can be guided, the second gripping means (16) can be fastened, and in which the at least one electrical contact of the electrode instrument (23) can be latched and/or electrically contacted, **characterized by** a contact body (17) as claimed in at least one of claims 1 through 10.

## Revendications

1. Corps de contact (17) pour un appareil à main électrochirurgical, en particulier un résectoscope (10), pour recevoir un guide optique tubulaire (18), pour fixer une unité de préhension (14) et pour coupler au moins un contact électrique d'un instrument à électrode (23) de l'appareil à main, **caractérisé en ce que en ce que** le corps de contact (17) présente une fente (32) parallèle à un alésage traversant (19) destiné à recevoir le guide optique (18) et parallèle à un axe longitudinal de l'appareil à main, la fente (32) s'étendant d'une paroi extérieure (35) du corps de contact (17) jusqu'à l'alésage (19), et le guide optique tubulaire (18) pouvant être guidé à travers la fente (32) dans l'alésage (19).

2. Corps de contact (17) pour un appareil à main électrochirurgical selon la revendication 1, **caractérisé en ce que** la fente (32) présente deux parois latérales (36, 37) parallèles ou formant un angle ou **en ce que** les parois latérales (36, 37) présentent une section transversale triangulaire, deux coins des parois latérales (36, 37) étant situés directement en vis-à-vis parallèlement l'un à l'autre.

3. Corps de contact (17) pour un appareil à main électrochirurgical selon l'une quelconque des revendications précédentes, **caractérisé en ce qu'**une section transversale du corps de contact (17) présente une forme annulaire, de préférence circulaire ou ovale, la fente (32) constituant une ouverture de la forme.

4. Corps de contact (17) pour un appareil à main électrochirurgical selon l'une quelconque des revendications précédentes, **caractérisé en ce qu'**un plan parallèle et centré entre les parois latérales (36, 37) coupe un axe central de l'alésage (19).

5. Corps de contact (17) pour un appareil à main électrochirurgical selon l'une quelconque des revendications précédentes, **caractérisé en ce qu'**une largeur de la fente (32) est inférieure au diamètre de l'alésage (19) .

6. Corps de contact (17) pour un appareil à main électrochirurgical selon l'une quelconque des revendications précédentes, **caractérisé en ce que** l'alésage (19) présente un diamètre de 3 mm à 6 mm, de préférence de 4 mm à 5 mm, en particulier de 4,6 mm, et la fente (32) une largeur de 3 mm à 4 mm, de préférence de 3,5 mm, le diamètre étant légèrement supérieur à un diamètre du guide optique tubulaire (18).

7. Corps de contact (17) pour un appareil à main électrochirurgical selon l'une quelconque des revendications précédentes, **caractérisé en ce qu'**un rapport entre une largeur de la fente (32), en particulier une distance entre les parois latérales (36, 37) de la fente (32), et un diamètre de l'alésage (19) est de 0,6 à 0,9, de préférence de 0,7 à 0,8, en particulier de 0,76.

8. Corps de contact (17) pour un appareil à main électrochirurgical selon l'une quelconque des revendications précédentes, **caractérisé en ce que** le corps de contact (17) est un chariot pour un résectoscope actif ou passif (10).

9. Corps de contact (17) pour un appareil à main électrochirurgical selon l'une quelconque des revendications précédentes, **caractérisé en ce que** le corps de contact (17) est fabriqué en matière plastique, en particulier en PTFE, PFA, un autre polymère fluoré ou en PEEK.

10. Corps de contact (17) pour un appareil à main électrochirurgical selon l'une quelconque des revendications précédentes, **caractérisé en ce que** le corps de contact (17) présente au moins un logement (27) pour un contact de l'instrument à électrode (23) et ce logement (27) est relié à au moins une douille à fiche (28), en particulier **en ce qu'**une douille à fiche (28) est intégrée dans le corps de contact (17).

11. Appareil à main électrochirurgical, en particulier un résectoscope (10), avec un instrument à électrode (23) qui présente une électrode (24) à une extrémité distale et au moins un contact électrique à une extrémité proximale avec une unité de préhension (14) constituée d'un premier moyen de préhension (15) et d'un second moyen de préhension (16), avec une tige tubulaire (12), qui est couplée par une extrémité proximale au premier moyen de préhension (15), avec un guide optique (18) destiné à recevoir une optique (29) et un corps de contact (17) à travers lequel le guide optique (18) peut être guidé, le deuxième moyen de préhension (16) peut être fixé et dans lequel l'au moins un contact électrique de l'instrument à électrode (23) peut être encliqueté et/ou mis en contact électrique, **caractérisé par** un corps de contact (17) selon au moins l'une quelconque des revendications 1 à 10.
